# EUROPEAN PATENT APPLICATION

(11) **EP 4 657 455 A1**
(43) Date of publication of application: **03.12.2025**
(21) Application number: 25179336.0
(22) Date of filing: 28.05.2025
(51) Int. Cl.: G16H 40/63

(54) **SYSTEMS, DEVICES, AND METHODS FOR MEDICAL DEVICE CONTROL**

(30) Priority: 29.05.2024 US 202463653156 P
(71) Applicant: Stryker Corporation, Portage, MI 49002-9711 (US)
(72) Inventor: KUMAR, Ashutosh, Portage, 49002 (US); MURRAY, Gavin Michael, Portage, 49002 (US); WONG, Vivian Wing Yan, Portage, 49002 (US)
(74) Representative: V.O.

(57) **Abstract**

A method for controlling medical devices includes receiving a user input to a user control device that is communicatively connected to a first medical device controller, the first medical device controller configured to control a first medical device; transmitting a message associated with the user input from the first medical device controller to a second medical device controller; and controlling, by the second medical device controller, based on the message, a second medical device communicatively connected to the second medical device controller.

## Description

### CROSS-REFERENCE TO RELATED APPLICATIONS

This application claims the benefit of U.S. Provisional Application No. 63/653,156, filed May 29, 2024, the entire contents of which are hereby incorporated by reference herein.

### FIELD

The present application relates generally to control of medical tools, and more specifically to systems and methods for controlling different medical devices with the same user control device.

### BACKGROUND

A medical room, such as an operating room, is often crowded with multiple user control devices for controlling different medical devices. For example, an operating room may include multiple foot switches, hand controls, and/or other user control devices that control different tools that a surgeon might use during a surgical procedure. The greater the number of user control devices, the greater the clutter, the longer the setup time, the longer the time needed for cleaning or sterilization, and the greater the cognitive burden on a user (e.g., a surgeon, medical staff, and the like) to remember which user control devices are mapped to which medical devices or the need to ask other staff to trigger an input. Sometimes, different medical devices can be controlled using the same type of user control device. However, such arrangements typically require personnel (e.g., a non-sterile staff member at the direction of a surgeon) to connect the user control device to a controller of another medical device, which may be time-consuming, imprecise, and prone to human error.

### SUMMARY

According to an aspect, methods, systems, and devices enable selective controlling of different medical devices using the same user control device. In a medical environment, such as an operating room, that includes multiple controllable medical devices that are controlled by multiple medical device controllers, a user control device connected to one of the medical device controllers can be used to control a medical device connected to a different medical device controller by associating the user control device with the medical device. For example, a central controller may determine which medical device with which to associate the user control device and may provide a mapping of user control device to medical device to each medical device controller. A medical device controller to which a user control device is connected may then communicate a user input made via the user control device to a medical device controller to which the associated medical device is connected. The medical device controller connected to the medical device then controls the medical device according to the user input to the user control device.

Determining which medical device with which to associate the user control device may be done automatically, such as by determining which medical device is being used, or is being prepared for use, by a user. This determination may be made, for example, via image processing that identifies the medical device in one or more images or video. The images or video may be generated by a medical imager, such as an endoscopic or open-field imager, that captures the medical device in use in a surgical field or by a camera system that captures a user holding the medical device. The medical device may be identified in the image or video data and the user control device may be associated with the medical device accordingly. Additionally, or alternatively, a user may manually select the mapping of the user control device to medical device, such as via a voice command, selection user interface, and/or input to the user control device.

According to an aspect, a method for controlling medical devices includes receiving a user input to a user control device that is communicatively connected to a first medical device controller, the first medical device controller configured to control a first medical device; transmitting a message associated with the user input from the first medical device controller to a second medical device controller; and controlling, by the second medical device controller, based on the message, a second medical device communicatively connected to the second medical device controller.

The method may include, prior to receiving the user input, associating the user control device with the second medical device. Associating the user control device with the second medical device may include receiving a user selection to associate the user control device with the second medical device. The user selection may be received via the user control device. The user selection may include a voice command, a gesture, an eye-tracking input, a virtual or augmented reality control, a button press, or an input to a touch input device. The touch input device may be a touch input device of a central controller that is communicatively connected to the first and second medical device controllers.

Associating the user control device with the second medical device may include determining use of the second medical device by a user. The use of the second medical device may be determined based on video or image data. Determining use of the second medical device by the user may include identifying the second medical device in the video or image data. The video or image data may include endoscopic video or image data capturing a surgical cavity within which the second medical device is positioned. The video or image data may capture the user holding the second medical device. The use of the second medical device may be determined based on sensor data generated by at least one sensor of the second medical device.

Associating the user control device with the second medical device may include transmitting from a central controller to at least the first medical device controller at least one instruction associating the user control device with the second medical device. The method may include displaying on at least one display an indication that the user control device is set to control the second medical device. The method may include displaying the indication that the user control device is set to control the second medical device along with endoscopic video or images.

According to an aspect, a system for controlling medical devices includes a first medical device controller configured to control a first medical device and communicatively connected to a user control device; and a second medical device controller configured to control a second medical device and communicatively connected to the first medical device controller, the first medical device controller comprising one or more processors and memory storing one or more programs for execution by the one or more processors, the one or more programs comprising instructions that, when executed by the one or more processors, cause the first medical device controller to: receive a user input via the user control device, and transmit a message associated with the user input to the second medical device controller, and the second medical device controller including one or more processors and memory storing one or more programs for execution by the one or more processors, the one or more programs comprising instructions that, when executed by the one or more processors, cause the second medical device controller to: receive the message associated with the user input, and control the second medical device based on the message associated with the user input.

The system may include a central controller communicatively connected to the first and second medical device controllers and configured to associate the user control device with the second medical device. The central controller may be configured to associate the user control device with the second medical device based on receiving a user selection to associate the user control device with the second medical device. The user selection may be received via the user control device. The user selection may include a voice command, a gesture, an eye-tracking input, a virtual or augmented reality control, a button press, or an input to a touch input device. The touch input device may be a touch input device of the central controller. The central controller may be configured to associate the user control device with the second medical device by determining use of the second medical device by a user. The central controller may be configured to determine the use of the second medical device based on video or image data. The central controller may be configured to determine use of the second medical device by the user comprises identifying the second medical device in the video or image data. The video or image data may include endoscopic video or image data capturing a surgical cavity within which the second medical device is positioned. The video or image data may capture the user holding the second medical device. The use of the second medical device may be determined based on sensor data generated by at least one sensor of the second medical device.

The central controller may be configured to associate the user control device with the second medical device by transmitting from the central controller to at least the first medical device controller at least one instruction associating the user control device with the second medical device. The central controller may be configured to display on at least one display an indication that the user control device is set to control the second medical device. The central controller may be configured to display the indication that the user control device is set to control the second medical device along with endoscopic video or images.

According to an aspect, a non-transitory computer readable storage medium stores instructions for execution by one or more processors of first and second medical device controllers to cause the first and second medical device controllers to perform any of the above methods.

According to an aspect, a method for controlling medical devices includes determining, by a central controller, an association of a user control device with one of a plurality of medical devices, wherein the plurality of medical devices are controlled by a plurality of medical device controllers communicatively connected to the central controller; and transmitting, by the central controller, instructions to the plurality of medical device controllers indicating the association of the user control device with the one of the plurality of medical devices so that a user input to the user control device controls the one of the plurality of medical devices.

Determining the association of the user control device with the one of the plurality of medical devices may include determining use of the medical device by a user. The use of the medical device may be determined based on video or image data. Determining use of the medical device by the user may include identifying the medical device in the video or image data. The video or image data may include endoscopic video or image data capturing a surgical cavity within which the medical device is positioned. The video or image data may capture the user holding the medical device. The use of the medical device may be determined based on sensor data generated by at least one sensor of the medical device. Determining the association of the user control device with the one of the plurality of medical devices may include receiving a user selection corresponding to selection of the one of the plurality of medical devices. The user selection may be received via the user control device. The user selection may include a voice command, a gesture, an eye-tracking input, a virtual or augmented reality control, a button press, or an input to a touch input device. The touch input device may be a touch input device of the central controller.

The method may include displaying on at least one display an indication of the association of the user control device with the one of the plurality of medical devices. The method may include displaying the indication of the association of the user control device with the one of the plurality of medical devices along with endoscopic video or images.

According to an aspect, a system includes one or more processors and memory storing one or more programs for execution by the one or more processors, the one or more programs comprising instructions that, when executed by the one or mor processors, cause the system to: determine an association of a user control device with one of a plurality of medical devices, wherein the plurality of medical devices are controlled by a plurality of medical device controllers; and transmit instructions to the plurality of medical device controllers indicating the association of the user control device with the one of the plurality of medical devices so that a user input to the user control device controls the one of the plurality of medical devices.

The system may include the plurality of medical device controllers. Determining the association of the user control device with the one of the plurality of medical devices may include determining use of the medical device by a user. The use of the medical device may be determined based on video or image data. Determining use of the medical device by the user may include identifying the medical device in the video or image data. The video or image data may include endoscopic video or image data capturing a surgical cavity within which the medical device is positioned. The video or image data may capture the user holding the medical device. The use of the medical device may be determined based on sensor data generated by at least one sensor of the medical device. Determining the association of the user control device with the one of the plurality of medical devices may include receiving a user selection corresponding to selection of the one of the plurality of medical devices. The user selection may be received via the user control device. The user selection may include a voice command, a gesture, an eye-tracking input, a virtual or augmented reality control, a button press, or an input to a touch input device.

The one or more programs may include instructions that cause the system to display on at least one display an indication of the association of the user control device with the one of the plurality of medical devices. The one or more programs may include instructions that cause the system to display the indication of the association of the user control device with the one of the plurality of medical devices along with endoscopic video or images.

According to an aspect, a non-transitory computer readable storage medium stores instructions for execution by one or more processors of a computing system to cause the computing system to perform any of the above methods.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1 illustrates an exemplary system that enables a user to control different medical devices with the same user control device;
FIG. 2 illustrates an exemplary method for controlling medical devices;
FIG. 3 illustrates an exemplary method for controlling medical devices that may include associating a user control device with one of a plurality of medical devices;
FIG. 4 illustrates an exemplary user interface for enabling a user to make a selection for associating a user control device with a medical device;
FIG. 5 illustrates an exemplary user interface displaying medical images or video via which a user may provide a selection for mapping a user control device with a medical device;
FIG. 6 illustrates an example of a central controller detecting the presence of a medical device in an endoscopic video frame;
FIG. 7A illustrates an exemplary method for setting up connections between a central controller and one or more medical device controllers that enables a user control device connected to one medical device controller to control a medical device connected to another medical device controller;
FIG. 7B illustrates an exemplary method for using a user control device connected to one medical device controller to control a medical device connected to another medical device controller;
FIG. 8 illustrates an exemplary mapping table that may be maintained by a central controller for mapping a user control device to a medical device; and
FIG. 9 illustrates an exemplary computing system.

### DETAILED DESCRIPTION

Reference will now be made in detail to implementations and examples of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Described herein are examples of methods, systems, and devices for enabling a user control device connected to one medical device controller to be used for controlling a medical device connected to a different medical device controller. Enabling the control of multiple different medical devices with one user control device may reduce the number of user control devices required in a medical environment, such as an operating room. This can reduce complexity, reduce set-up time, enable faster reconfiguration within the same medical procedure or between medical procedures, reduce cleaning and sterilization, and reduce the risk of a user error associated with connection of user control devices to medical devices.

A medical environment, such as an operating room, may include multiple controllable medical devices, such as one or more handheld cutters, burs, drills, etc., that may be used during a medical procedure on a patient. Different medical devices may be connected to and controlled by different medical device controllers. A user control device, such as a foot pedal or handheld controller, may be connected to one of the medical device controllers but may be associated with a medical device connected to a different medical device controller so that user inputs to the user control device control the medical device.

A central controller communicatively connected to the plurality of medical device controllers may determine which medical device that the user control device should be associated with (mapped to) and may provide the mapping to the medical device controllers. Based on the mapping information provided by the central controller, a medical device controller to which the user control device is connected may handle a user input to the user control device by transmitting a message (directly or via one or more intermediate components, such as the central controller) to the medical device controller to which the associated medical device is connected, with the message including information associated with the user input. The receiving medical device controller may then control its connected medical device accordingly.

The central controller may determine the medical device to which a user control device should be mapped automatically. The central controller may automatically determine the mapping by determining that a user is using, or is preparing to use, a particular medical device. Use of the medical device may be determined by identifying the medical device in one or more images or video. The images or video can be generated by a medical imager, such as an endoscopic imager or open-field imager that captures the medical device in the surgical field. The images or video can be generated by an imaging system that captures the user holding the medical device, such as a point-tilt-zoom (PTZ) camera, in-light camera, and/or one or more navigation system cameras. Additionally, or alternatively, use of the medical device can be automatically determined via one or more sensors of the medical device. Additionally, or alternatively, a user may manually select the mapping via any suitable user interface, such as via a touch panel, a voice command system, or a suitable user input of the user control device.

Mapping of a user control device to a medical device may be updated at any time so that, for example, the user control device can be used to control one medical device for one part of a medical procedure and can be used to control a different medical device for a different part of the medical procedure. The updated mapping can occur automatically, such as when a different medical device is identified in one or more images or video or via a user's manual selection.

In the following description of the various examples, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units, but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like refer to the action and processes of a computer system, or similar electronic computing device, that manipulates and transforms data represented as physical (electronic) quantities within the computer system memories or registers, or other such information storage, transmission, or display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application-specific integrated circuits (ASICs), or any type of media suitable for storing electronic instructions, and each connected to a computer system bus. Furthermore, the computing systems referred to in the specification may include a single processor or may be architectures employing multiple-processor designs, such as for performing different functions or for increased computing capability. Suitable processors include central processing units (CPUs), graphical processing units (GPUs), field programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present disclosure as described herein.

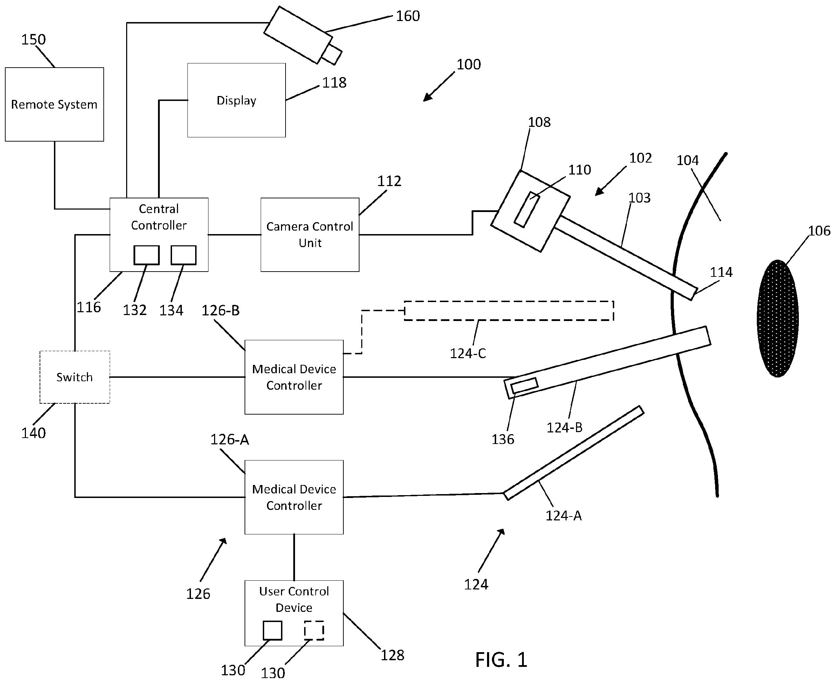

FIG. 1 illustrates an exemplary system 100 that enables a user to control different medical devices with the same user control device. System 100 may be used in a medical environment, such as an operating room. System 100 includes an endoscopic imaging device 102 for insertion into a surgical cavity 104 for imaging tissue 106 within the surgical cavity 104 during a medical procedure. The endoscopic imaging device 102 may include an endoscope 103 that extends from an endoscopic camera head 108 that includes one or more imaging sensors 110. Light reflected and/or emitted (such as fluorescence light emitted by fluorescing targets that are excited by fluorescence excitation illumination light) from the tissue 106 is received by the distal end 114 of the endoscope 103. The light is propagated by the endoscope 103, such as via one or more optical components (for example, one or more lenses, prisms, light pipes, or other optical components), to the camera head 108, where it is directed onto the one or more imaging sensors 110. One or more filters (not shown) may be included in the endoscope 103 and/or camera head 108 for filtering a portion of the light received from the tissue 106 (such as fluorescence excitation light). While the example implementation of system 100 illustrated in FIG. 1 is configured for endoscopic imaging, this is merely exemplary. It should be understood that system 100 can include any type of medical imaging system, including an exoscopic imaging system, a microscopic imaging system, an open-field imaging system, or may not have any imaging system at all. Although the discussion within often refers to system 100 as including a surgical system or surgical components, other examples of system 100 are non-surgical. For example, system 100 may include a non-surgical open-field imaging system, such as for imaging a subject without any surgical or invasive steps (e.g., through-the-skin fluorescence imaging for determining perfusion of peripheral tissue).

The one or more imaging sensors 110 generate pixel data that can be transmitted to a camera control unit 112 that is communicatively connected to the camera head 108. The camera control unit 112 generates a video feed from the pixel data that shows the view of the endoscopic imaging device 102. The video feed can be transmitted to a central controller 116 for further image processing, storage, display, and/or routing to one or more remote computing systems 150 such as a cloud computing system. The video feed or portions thereof can be transmitted to one or more displays 118 from the central controller 116, for visualization by medical personnel, such as by a surgeon for visualizing the surgical cavity 104 during a surgical procedure on a subject. The one or more displays can include monitors, touchscreens, head-mounted displays, including virtual reality and mixed reality displays, boom-mounted displays, wall-mounted displays, etc.

The central controller 116 may receive and process video and/or image data generated by imaging devices other than the endoscopic imaging device 102. For example, the central controller 116 may receive video and/or image data generated by one or more camera systems 160 that capture video and/or images from outside of the body of the subject. The video and/or images generated by the one or more camera systems 160 may capture the use of one or more tools by a user performing a surgical procedure on the subject. The one or more camera systems 160 can include, for example, a PTZ camera, an in-light camera, and/or a stereoscopic camera that generates three-dimensional image data.

System 100 may include multiple medical devices 124, such as medical device 124-A and medical device 124-B, which may be available for use during the surgical procedure on the subject. The medical devices 124 can include devices used during endoscopic surgical procedures and/or devices used during open surgical procedures. Examples of medical devices 124 include burs, resection devices, RF probes, cutters, and ultrasonic aspirators. The medical devices 124 can include medical devices that are controlled by medical device controllers 126. One or more of the medical device controllers can be embodied as a console (e.g., a dedicated console including circuitry and/or programming configured to control one or more functions of a medical device, a user interface to display one or more device settings to a user, and one or more user input devices for receiving user inputs associated with control of the medical device). In the example illustrated in FIG. 1, medical device 124-A is controlled by medical device controller 126-A and medical device 124-B is controlled by medical device controller 126-B. For example, medical device 124-A may be a drill, and medical device controller 126-A may control one or more functions of the drill, such as a start/stop function, a speed function, a direction function, etc. Medical device 124-B may be an ultrasonic aspirator, and medical device controller 126-B may control the ultrasonic power, suction, and/or irrigation of the ultrasonic aspirator. A medical device 124 and its medical device controller 126 may be separate components that are communicatively connected, as depicted in FIG. 1, or a medical device may have a built-in medical device controller. Optionally, a medical device controller 126 may be configured to control multiple medical devices 124. For example, medical device controller 126-B may control medical device 124-B and medical device 124-C. A medical device controller 126 may control multiple of the same type of medical device and/or multiple medical devices of different types.

The medical devices 124 can include surgical and/or non-surgical devices. Reference within to surgical devices is merely exemplary as devices controlled according to the principles described herein can be any medical device, including non-surgical devices. Examples of non-surgical devices that can be included in system 100 and controlled according to the principles described herein include non-invasive imaging systems (such as open-field fluorescence imaging systems, ultrasonic imaging systems, and in-light cameras), illumination systems (such as hand-held light sources and boom-mounted medical lights), and laser treatment devices.

System 100 may include one or more user control devices 128 that a user can use to control medical devices 124. The user control device 128 can include any device that can receive inputs from a user. Examples of user control devices 128 include foot pedals, hand-held controls, touchscreen devices, and joysticks. The user control device 128 may include one or more user input devices 130 that a user may interact with to control a medical device 124. The user input devices 130 can include one or more buttons, switches, touch input devices, knobs, levers, etc. The user control device 128 may be communicatively connected to one of the medical device controllers 126 and may transmit signals or messages to the medical device controller 126 to which it is connected in response to receiving a user input. For example, a user may press a button of the user control device 128 and the user control device 128 may respond by sending an analog signal or a digital message to the medical device controller 126 to which the user control device 128 is connected. As explained further below, system 100 may be configured so that the same user control device 128 can be used to control different medical devices 124 so that the user can control multiple medical devices 124 without requiring the use of multiple user control devices and without requiring a user to disconnect the user control device 128 from the medical device controller 126 to which it is connected and to reconnect the user control device 128 to a different medical device controller 126. Thus, for example, the user control device 128 connected to medical device controller 126-A can be used to control medical device 124-B without having to connect the user control device 128 to medical device controller 126-B.

Although FIG. 1 depicts a single user control device 128, system 100 may include any number of user control devices 128. Different user control devices 128 can be connected to different medical device controllers 126 and/or to the same medical device controller 126. A user control device 128 may connected to a medical device controller 126 in a wired manner or wirelessly. Optionally, a user control device 128 may be connected to the central controller 116 with a wired or wireless connection.

The medical device controller 126 may be communicatively connected to the central controller 116, either directly or through one or more network communication components 140 (such as a switch or router) and/or networks. The central controller 116 may control how one or more user control devices 128 are mapped to the medical devices 124 so that a user input to a particular user control device 128 controls the appropriate medical device 124. The central controller 116 may include one or more user input devices 132 for enabling a user to make selections associated with the operation of the central controller 116. The central controller 116 may include a display screen 134 for displaying aspects of operation of the central controller 116.

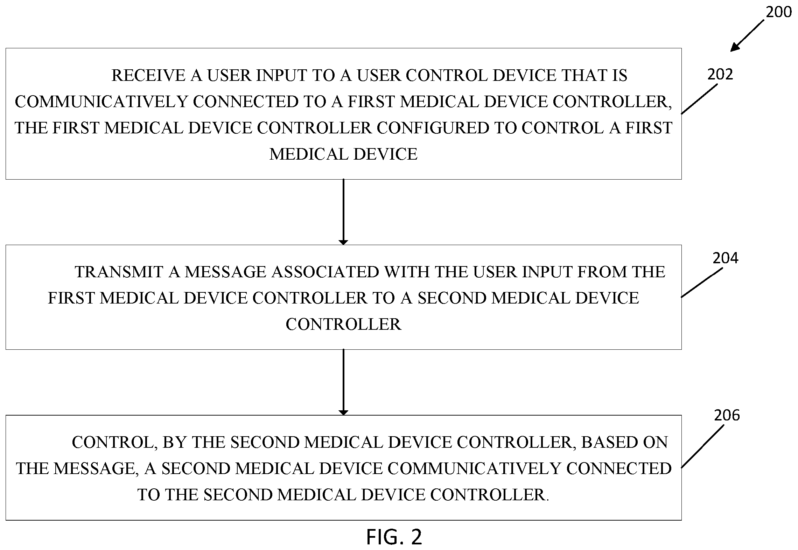

FIG. 2 illustrates an exemplary method 200 for controlling medical devices. Method 200 may be used to selectively control different medical devices using the same user control device. Method 200 may be performed by one or more computing systems. Method 200 may be performed by one or more components of system 100 of FIG. 1. For example, one or more steps of method 200 may be performed by one or more medical device controllers 126, one or more medical devices 124, central controller 116, and/or one or more user control devices 128.

At step 202, a user input is received at a user control device. For example, with reference to FIG. 1, the user control device 128 may receive a user input via the user input device 130 (e.g., a button press). The user control device may be connected to a first medical device controller that is configured to control a first medical device. For example, in FIG. 1, the user control device 128 is connected to medical device controller 126-A, which is configured for controlling medical device 124-A.

At step 204, a message associated with the user input is transmitted from the first medical device controller to a second medical device controller. For example, a message may be transmitted from medical device controller 126-A to medical device controller 126-B. The first and second medical device controllers are communicatively connected, either directly or through one or more networking components and/or one or more networks. For example, with respect to system 100, the medical device controller 126-A may transmit a message to medical device controller 126-B via network communication component 140. In some variations, the message may be routed through the central controller 116.

The message may include an indication of the user input to the user control device. For example, the message may indicate that a button press was received by the user control device, or if the user control device has multiple buttons, the message may indicate which button was pressed. Additionally, or alternatively, the message may include a command associated with the user input. For example, the user may press a button of the user control device that corresponds with increasing speed of a cutter and the message may include an increase speed command. The message may be generated by the user control device, by the first medical device controller, or by a combination of the user control device and the first medical device controller. For example, with reference to system 100, the user control device 128 may generate a message based on a user input to the user control device 128 and may transmit the message to the medical device controller 126-A. The medical device controller 126-A may then pass the message (with or without modifying the message such as to include or remove routing information) to medical device controller 126-B. Alternatively, the medical device controller 126-A may generate the message based on one or more inputs from the user control device 128. For example, the user control device 128 may transmit one or more analog and/or digital signals associated with the user input, the medical device controller 126-A may then determine the type of user input based on the one or more signals and may generate a message that includes an indication of the user input.

The message associated with the user input that is transmitted from the first medical device controller to a second medical device controller may include information identifying the second medical device and/or the user control device. This information may be used by one or both of the first medical device controller and the second medical device controller for determining that the message includes a control command for the second medical device. For example, the first medical device controller and the second medical device controller may be connected via an IP network and the message may include an IP address of the second medical device.

At step 206, the second medical device controller controls a second medical device that is communicatively connected to the second medical device controller based on the message that the second medical device controller received from the first medical device controller. For example, with reference to FIG. 1, medical device controller 126-B may control medical device 124-B based on the message that it receives from medical device controller 126-A, the message being associated with a user input to the user control device 128 connected to the medical device controller 126-A. The second medical device controller may parse the message to determine how to control the second medical device. For example, the second medical device controller 126-B may determine that the message includes information corresponding with an increase speed command for medical device 124-B and may increase the speed of the medical device (e.g., a rotational speed of a cutter).

The first medical device controller may communicate the message to the second medical device controller based on a predetermined association of the user control device with the second medical device that is known to the first medical device controller. Additionally, the control of the second medical device by the second medical device controller may also be based on the predetermined association of the user control device with the second medical device, which is known to the second medical device controller. Such a predetermined association may enable the first medical device controller to determine that the user input to the user control device is not for controlling the first medical device (which first medical device controller controls) but, rather, is intended for controlling the second medical device. Similarly, the second medical device controller may determine based on its knowledge of the predetermined association that it should use the information in the message for controlling the second medical device (which it controls). The association of the user control device to a medical device may be changeable so that the user control device can be used to control a different medical device than the second medical device. For example, with reference to system 100 of FIG. 1, the user control device 128 can be associated with any one of medical device 124-A, 124-B, and 124-C. The association may be switched during a medical procedure on a subject so that a user can switch between controlling different medical devices as desired. For example, the user control device can be used to control the second medical device while the user is using the second medical device and can be switched to controlling the first medical device when the user prepares to use, or begins using, the first medical device.

The association of the user control device with a particular medical device (also referred to herein as a mapping) may be controlled via a central controller. For example, with reference to system 100, the association of the user control device 128 with one of the medical devices 124-A, 124-B, and 124-C can be controlled via central controller 116. Controlling the association of the user control device with a particular medical device via the controller may include a controller determining the association of the user control device with the particular medical device and one or more of the medical device controllers confirming the determined association of the user control device with the particular medical device.

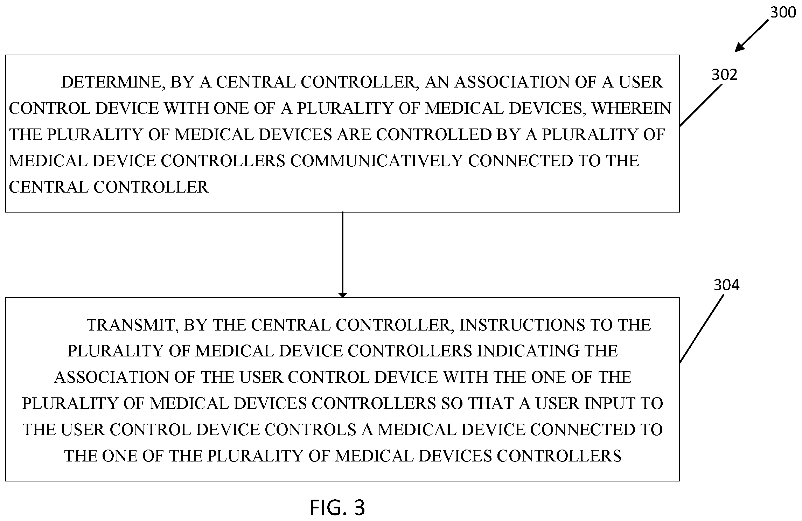

FIG. 3 illustrates an exemplary method 300 for controlling medical devices that may include associating a user control device with one of a plurality of medical devices. Method 300 may be performed at least in part by a central controller, such as central controller 116 of system 100. The central controller can be provided as a single computing system, such as central controller 116 of FIG. 1, or can include multiple communicatively coupled computing systems, such as a combination of central controller 116 and one or more remote computing systems 150. As such, different functions of the central controller can be implemented by different computing systems that are communicatively coupled to one another. For example, image frame extraction from video can be implemented by a local computing system that is located in an operating room and one or more machine learning models can be implemented by one or more remote computing systems, such as a cloud computing system, which receive the image frames from the local computing system and return information regarding detected features to the local computing system. The central controller generally includes one or more processors, which can include one or more CPUs, one or more GPUs, one or more TPUs, and/or one or more FPGAs.

Method 300 may be performed together with method 200 of FIG. 2. For example, method 300 may be performed prior to step 202 of method 200 for associating the user control device with the second medical device and/or after step 206 of method 200 for switching association of the user control device from the second medical device to the first medical device or some other medical device.

At step 302, a central controller determines an association of a user control device with one of a plurality of medical devices. For example, with reference to system 100 of FIG. 1, the central controller 116 may determine an association of the user control device 128 with medical device 124-B such that user inputs to user control device 128 control medical device 124-B. The medical devices are controlled by medical device controllers that are communicatively connected to the central controller. For example, with reference to system 100 of FIG. 1, medical device 124-B is controlled by medical device controller 126-B, which is communicatively connected to the central controller 116.

The association of the user control device with one of a plurality of medical devices may be determined in a number of different ways. In some examples, a user may select the association. The user selection may be made manually via a user input to the central controller or a device communicatively connected to the central controller. The user selection may be made by the user of the user control device or by another user. For example, a surgeon may instruct another medical personnel in an operating room to change the selection of the medical device that is being controlled by a user control device available to the surgeon. The user selection may include a voice command captured by one or more microphones, such as one or more microphones (not shown) disposed on camera system 160 and/or central controller 116. Additionally, or alternatively, the user selection may include a gesture, an eye-tracking input, and/or a virtual or augmented reality control (e.g., captured by camera system 160). Additionally, or alternatively, the user selection may include a button press (e.g., received by a button of user control device 128 and/or central controller 116) and/or an input to a touch input device (e.g., received by a touch input device of user control device 128 and/or central controller 116). Generally, the user selection may be made via any available and suitably configured user input capability.

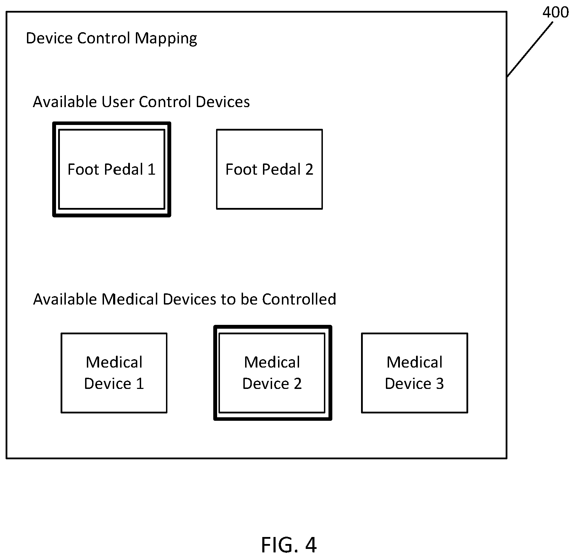

The central controller may display a user interface that facilitates user selection of the association of the user control device with one of a plurality of medical devices. FIG. 4 illustrates an exemplary user interface 400 for facilitating user selection of the association of a user control device with a medical device. User interface 400 may be displayed on a display of the central controller and/or a display communicatively connected to the central controller. For example, user interface 400 may be displayed on a display screen 134 (e.g., a touchscreen display) of central controller 116 and/or on display 118.

User interface 400 may display the available user control devices (which may be just a single user control device if only one user control device is available) that may be selected, which in the illustrated example includes Foot Pedal 1 and Foot Pedal 2. A user may provide an input selecting Food Pedal 1 or Foot Pedal 2 (in the illustrated example, Foot Pedal 1 has been selected). For example, the user may provide an input via a touch input device (e.g., a touchscreen or touch pad) of the central controller, via a voice command detected by a voice command system (such as a voice command system that captures voice commands using one or more microphones (not shown) disposed on camera system 160 and/or central controller 116), via a gesture detected by a gesture control system (such as a gesture control system that uses images captured by camera system 160), via an eye-tracking input detected by an eye-tracking system (such as an eye-tracking system that uses images captured by camera system 160), via virtual or augmented reality control, via a remote control, via a press of a button or touch pad or other button of the central controller, or via input to some other user input device communicatively connected to the central controller. If only one user control device is available, then it may be selected by default. The user providing the selection input may not be the user of the control device. For example, a nurse or other non-sterile user may be instructed by a sterile surgeon to enter the selection. The non-sterile user may use a touch interface communicatively connected to the central controller to map the desired medical device to the desired user input device. The touch panel may be, for example, a touch panel of the central controller or a touch panel communicatively connected to the central controller, such as a wall mounted touch panel, a cart mounted touch panel, or a touch panel at a nurse's station.

User interface 400 may display the available medical devices that may be controlled by the selected user control device. In the illustrated example, user interface 400 displays Medical Devices 1-3 as available for mapping to the available user control devices, with Medical Device 2 being the device selected for control by Foot Pedal 1. Based on the user's selection of Foot Pedal 1 and Medical Device 2, the central controller determines that Foot Pedal 1 should be associated with Medical Device 2 so that inputs to Foot Pedal 1 control the operation of Medical Device 2. The user may select a mapping for Foot Pedal 2 (and any other available user control devices) by selecting Foot Pedal 2 in the user interface 400 and then selecting the desired medical device. Optionally, a medical device already associated with a user control device may be unavailable for selection so that multiple user control devices are not mapped to the same medical device. Optionally, the medical device to be controlled may be selected first before the user control device is selected. Additionally, or alternatively, to providing touch selections, a user may type in a command, such as "Foot Pedal 1 to Medical Device 2."

Optionally, a user may provide a selection associated with mapping a user control device to a medical device using the user control device itself. For example, a user may provide one or more button presses to a foot pedal to select the medical device to be controlled by the foot pedal. A user input to a suitable input device of a user control device (e.g., a button press to a foot pedal) may result in a medical device selection message being sent to the central controller. The central controller may determine which user control device is associated with the message and, thereby, which user control device is being mapped by the user. With reference to FIG. 4, the central controller may include in user interface 400 an indicator of a selection of the user control device that originated the message, thereby indicating to the user that the user has made a selection for mapping that user control device. For example, a user button press to Foot Pedal 1 may result in Foot Pedal 1 being highlighted in the user interface 400. In the example shown in FIG. 4, an icon representing Foot Pedal 1 is encompassed with a border indicating that the user has made a selection for mapping Foot Pedal 1. The central controller may cycle between the available medical devices based on the medical device selection message. For example, with reference to FIG. 4, a button press to Foot Pedal 1 may result in the selection of Medical Device 2 switching to Medial Device 3. Subsequent button presses to Foot Pedal 1 may cycle to Medical Device 1, then to Medical Device 2, and so on. The central controller may update the mapping of the user control device to the medical device upon receiving the medical device selection message.

A user may provide a selection for mapping a user control device to a medical device using voice control. The central controller may include voice control functionality or may be communicatively connected to a voice control system. A user can provide a voice command that may be captured by any suitable microphone or microphone array in the vicinity of the user. The voice command may be parsed by the central controller or by the connected voice control system. The central controller may then determine the mapping of the user control device accordingly. The voice command may directly indicate the mapping-such as "Map foot pedal 1 to medical device 2"-or may provide a command for cycling through mappings-such as "Cycle to next available medical device."

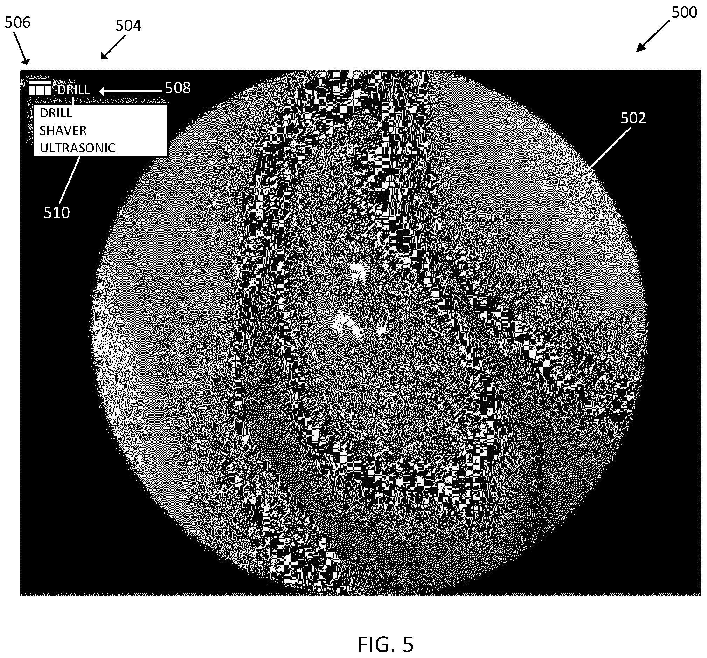

As noted above, one or more displays (e.g., display 118 of system 100) may display a user interface that facilitates user selection of the mapping of a user control device with a medical device. Optionally, medical images or video being generated by a medical imager used for treatment of the subject may be displayed as part of or along with a user interface that facilitates user selection of the mapping of a user control device with a medical device. For examples, a user interface that facilitates user selection of the mapping of a user control device with a medical device may be displayed proximate to the medical images or video, which may provide optimized visibility and ergonomic support by not requiring a user to look at additional consoles and/or displays for selecting the mapping. An example of such a user interface is illustrated in FIG. 5. User interface 500 displays an endoscopic video feed 502 that is captured, for example, by an endoscopic imager being used by the user (or an associated user). The user interface 500 may include a graphical and/or textual indicator 504 indicating the mapping of a user control device to a medical device. In the illustrated example, an icon 506 indicates the user control device (a foot pedal in the illustrated example) and text 508 indicates the medical device mapped to the user control device (a drill in the illustrated example). The user interface 500 may facilitate switching of the mapping of a user control device to a medical device. In the illustrated example, the user has activated a menu 510 that provides a list of medical devices that can be mapped to the user control device. The user may make a selection from the list using any suitable user input, including a voice command or a press to a button of a camera head, such as camera head 108 of system 100 of FIG. 1. The user may activate the menu 510 and make the selection from the list via any suitable user input, such as a voice command, a button press to a camera head (such as camera head 108 of system 100 of FIG. 1), a user input to a suitable input device of the user control device (e.g., user input device 130 of user control device 128), or an input to a touch screen display that displays the user interface (e.g., a touchscreen variation of display screen 134 of central controller 116 and/or a touchscreen variation of display 118).

Step 302 may include a central controller automatically determining an association of a user control device with one of a plurality of medical devices. The central controller may automatically determine the association of a user control device with a given medical device based at least in part on determining that the medical device is being used or is being prepared for use.

The use of the medical device may be determined by the central controller based on one or more sensors associated with the medical device detecting that the medical device is being handled. The one or more sensors can include, for example, one or more accelerometers or inertial measurement units, one or more touch sensors, and/or one or more pressure sensors. In response to one or more of the sensors detecting handling of the medical device (e.g., movement of the medical device and/or touch of the medical device), a communication may be transmitted from a medical device controller associated with the medical device to the central controller indicating that the medical device is being handled. The central controller may determine that the medical device is being handled based on the communication and may automatically map a user control device to the medical device. For example, with reference to FIG. 1, medical device 124-B may include at least one sensor 136 (e.g., an accelerometer, an inertial measurement unit, or a touch sensor) that detects when medical device 124-B is being moved (e.g., when its motion is above a predetermined threshold of motion associated with manipulation by a user). Although FIG. 1 illustrates one sensor 136, medical device 124-B can include any number of sensors that individually, or in any combination, can detect handling of the medical device 124-B by a user. In response to sensor 136 detecting movement of medical device 124-B, a communication may be transmitted from medical device controller 126-B to the central controller 116 indicating that medical device 124-B is being moved. The central controller 116 may then automatically map a user control device 128 to medical device 124-B. Optionally, a prompt may be provided to the user requesting confirmation of the automatically determined mapping.

The central controller may automatically determine the association of a user control device with a given medical device based on image data (video or image data). The central controller may be configured to identify a medical device in one or more images or video frames and may determine based at least in part on the identification of the medical device in the one or more images or video frames that the medical device is being used or is being prepared for use. The central controller may analyze the one or more images or video frames using one or more machine learning models to determine the presence of features in the image frames that correspond with a given medical device. The central controller receives image data from an imaging system, such as from camera control unit 112 of FIG. 1. The central controller may implement one or more classifiers for determining whether one or more medical devices are present in the image data. The one or more classifiers can each generate classification output data indicating the presence or absence of features (e.g., a medical device and/or one or more features of a medical device) in the one or more image frames. The central controller may make determinations regarding the presence or absence of features in the image data by comparing the classification output data generated by the one or more classifiers to predetermined thresholds. For example, the central controller may determine that a given feature is present in one or more image frames when classification output data generated by a classifier is above a predetermined threshold. The classification output data generated by the one or more classifiers may comprise one or more inference values. Classification output data may be provided as output to an external system for the external system to make determinations regarding the presence or absence of features in the one or more image frames by comparing the classification output data generated by the one or more classifiers to predetermined thresholds.

The central controller can be configured to determine the presence of one or more medical devices in a given image or video frame. For example, the one or more classifiers can be configured to determine the presence or absence of one or more cutters, one or more burs, one or more RF probes, and/or one or more drill bits. The central controller may include multiple classifiers, each classifier configured to determine the presence of a different type of medical device or may include a single classifier that can determine the presence of multiple different types of surgical tools. For instance, the central controller can include a first classifier configured to determine if a cutter is present in the image data and a second classifier configured to determine if a bur is present in the image data, or the central controller can include a single classifier that can determine if a cutter and a bur is present in an image or video frame.

The image or video frames used by the central controller to determine the use of a medical device may be captured by a medical imager being used during treatment of the subject, such as an endoscopic imager or an open-field imager. For example, with reference to FIG. 1, video or image data captured by endoscopic imaging device 102 may be used for determining that a medical device is being used. The central controller 116 may receive the video or image data via camera controller unit 112 and may analyze the video or image data to identify the presence of medical device 124-B in the field of view of the endoscopic imaging device 102, indicating to the central controller 116 that medical device 124-B is being used. Central controller 116 may then map the user control device 128 to medical device 124-B. If medical device 124-B were removed from within the field of view of the endoscopic imaging device 102 (e.g., removed from the surgical cavity 104) and medical device 124-A were inserted into the surgical cavity 104 within the field of view of the endoscopic imaging device 102, then the central controller 116 may detect the presence of medical device 124-A in the endoscopic video or images and may change the mapping of the user control device 128 to be mapped to medical device 124-A. Optionally, a prompt may be provided to the user requesting confirmation of the automatically determined mapping.

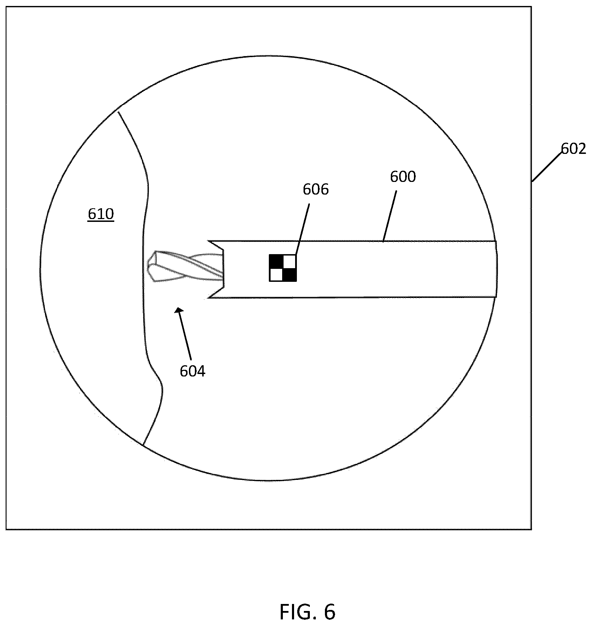

FIG. 6 illustrates an example of the central controller detecting the presence of a medical device 600 in an endoscopic video frame 602 capturing tissue 610 of a subject. The central controller may detect the presence of medical device 600 in the endoscopic video frame 602 and may determine that the type of the medical device 600 is a drill, such as based on the unique visual characteristics of the drill bit tip 604 of the drill relative to other types of medical devices that the central controller is configured to detect. Additionally, or alternatively, the medical device 600 may include a visual indicator 606 that is unique to that type of medical device 600 that can be detected by the central controller for determining the presence of the medical device 600 in the endoscopic video frame 602. The visual indicator 606 can be a fiducial marker, such as a QR code, and ArUco marker, a barcode, or any other indicator that is unique to the medical device 600 and can be detected by the central controller.

The image or video frames used by the central controller to determine the use of a medical device may be captured by an imaging system located outside of the subject. For example, with reference to FIG. 1, system 100 may include a camera system 160 that may image a scene that is external to the subject. The camera system 160 may include, for example, one or more room cameras (such as one or more PTZ cameras), one or more in-light cameras, or one or more 3D cameras (such as one or more 3D cameras of an optical surgical navigation system). The central controller 116 may receive the video or image data generated by the camera system 160 and may analyze the video or image data to identify the presence of a medical device 124 in the field of view of the camera system 160 indicating to the central controller 116 that the medical device 124 is being used or is being prepared for use. For example, a user may hold up a medical device to be in the field of view of a 3D camera system that may utilize two optical paths and a series of IR sensors to map a 3D image of the medical device. Using predetermined information for medical device shapes, the central controller or camera system may identify the tool by comparing its shape and/or size to the predetermined information to determine that the medical device 124 is being used or is being prepared for use. Central controller 116 may then map the user control device 128 to the identified medical device 124. In some variations, the image or video frames used by the central controller to determine the use of a medical device may be captured by an open-field imaging system, such as a hand-held open-field imager.

The central controller may be configured to not only identify the presence of the medical device in the image or video frames but also determine that the medical device is being grasped by a user. For example, one or more classifiers of the central controller may be configured for detecting a user's hand and/or the user's hand grasping the medical device. The central controller may be configured to determine that the user is using or preparing to use medical device only when identifying the presence of a medical device and that the medical device is being grasped, which may be useful when medical devices that are not being used are within a field of view of the camera system.

The central controller may be configured to determine who among a plurality of users is using an identified medical device and may map one of a plurality of user control devices to the identified medical device. For example, camera system 160 may generate images that capture a first user positioned on a first side of a subject and a second user positioned on a second side of the subject, and the central controller 116 may be configured to differentiate between a medical device grasped by the first user and a medical device grasped by the second user (e.g., based on the location of the medical device and/or user's hand grasping the medical device in the image). If the central controller 116 determines that the first user is grasping a particular medical device, the central controller 116 may map the particular medical device with a user control device 128 located on the first side of the subject so that the first user can use the user control device 128 located on the same side of the subject to control the particular medical device. Similarly, if the central controller 116 determines that the second user is grasping another medical device, the central controller 116 may map the other medical device with a user control device 128 located on the second side of the subject. Optionally, a prompt may be provided to the user requesting confirmation of the automatically determined mapping.

Optionally, the central controller may not itself determine that a medical device is being used (or is being prepared for use and/or being used by a particular user) but, instead, is communicatively connected to a system that makes that determination and notifies the central controller that a particular medical device is being used or is being prepared for use. For example, camera system 160 of FIG. 1 may include imaging processing capability that utilizes a suitable image processing algorithm, such as a suitable machine learning algorithm, to determine that a medical device is being used or is being prepared for use. The camera system 160 then communicates its determination to central controller 116, which maps the user control device 128 to the identified medical device 124 (with or without first requesting confirmation of the mapping from a user).

The central controller may determine the use or preparation for use of multiple different medical devices at the same time and may be configured to determine the mapping of a user control device to one of the multiple different medical devices based on one or more criteria. The predefined criteria can be associated with maintaining a current mapping over changing the mapping. For example, while a first medical device is being used (and a user control device is mapped to the first medical device), the central controller may determine that a second medical device being prepared for use or is now being used, and the central controller may maintain mapping of a user control device to the first medical device and not switch the mapping of the user control device to the second medical device (e.g., until the first medical device is no longer in use). The predefined criteria may be associated with preferring particular methods of determining use or preparation for use over other methods. For example, the central controller may determine use of a first medical device based on data from a motion sensor of the first medical device and may determine use of a second medical device based on identifying a user grasping the second medical device and may map a user control device to the first medical device based on predefined criteria that gives preference to motion sensor-based use determinations over imaging-based use determinations. The predefined criteria may be associated with preferring a particular user or class of user over other users or class of users. For example, the central controller may determine that a surgeon is holding a first medical device and that a medical staff member is holding a second medical device (e.g., using face recognition on images or video captured by camera system 160) and may map a user control device to the first medical device based on predefined criteria that assigns preference to surgeons over staff members.

The central controller may determine mapping based on a phase of a medical procedure. For example, the central controller may determine that a current or next phase in a medical procedure requires suction and may map a foot switch to a suction pump. The central controller may determine the phase of the medical procedure in various ways, such as by detecting activity associated with a particular phase in images or video (e.g., images or video captured by camera system 160) and/or by a user advancing through a procedure checklist provided by the central controller or system communicatively connected to the central controller.

In any of the above examples automatic mapping of a user control device to a medical device, a prompt may be provided to the user requesting confirmation of the automatically determined mapping. Optionally, automatic mapping of a user control device to a medical device according to any of the methods described above may be selectively activated and deactivated by a user. For example, a user may activate an automatic mapping mode during which the central controller determines mapping automatically (according to any of the methods or combination of methods described above) and may deactivate the automatic mapping mode such that any change in mapping requires manual selection. During the automatic mapping mode, the central controller may request user confirmation of any automatically determined mapping (which may be user-configurable).

Returning to FIG. 3, at step 304 of method 300, the central controller transmits at least one instruction to the plurality of medical device controllers indicating an association of the user control device with a particular medical device determined in step 302 so that a user input to the user control device controls that particular medical device. The instruction can include information associated with the medical device controller to which the particular medical device is connected and/or information associated with the particular medical device. For example, the instruction can include an IP address of the medical device controller to which the particular medical device is connected and/or an IP address associated with the particular medical device.

Method 300 may include one or more set-up steps that enable the central controller to know what user control devices and medical devices are available for mapping. Each medical device controller communicatively connected to the central controller may send a command to the central controller that identifies the medical device(s) connected to the medical device controller and/or the user control device(s) connected to the medical device controller. This communication can occur at any time and may occur in response to a medical device or user control device being connected to a given medical device controller. The command sent to the central controller from a medical device controller may indicate the type of medical device or user control device connected to the medical device controller. The central controller may add the user control device or medical device to a table of devices available for mapping.

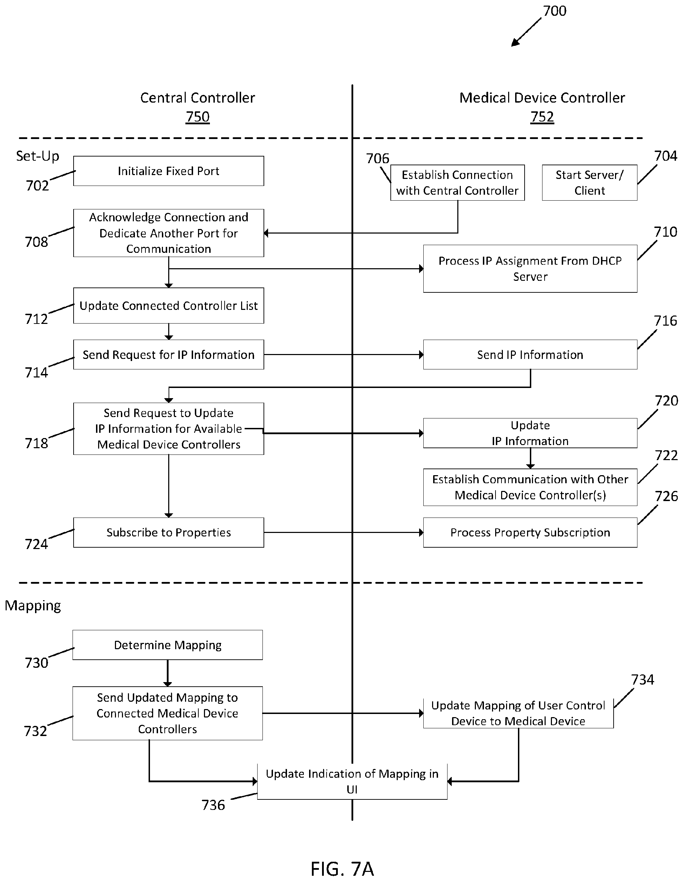

FIG. 7A illustrates an exemplary method 700 for setting up connections between a central controller 750 and one or more medical device controllers 752 that enables a user control device connected to one medical device controller to control a medical device connected to another medical device controller. Although only one medical device controller 752 is illustrated in FIG. 7A, it is to be understood that similar steps can be repeated for any number of medical device controllers. Central controller 750 may correspond to central controller 116 of system 100 of FIG. 1 and medical device controller 752 may correspond to medical device controller 126 of system 100 of FIG. 1.

At step 702, the central controller 750 initializes a fixed communication port that one or more medical device controllers may use to communicate with the central controller. This may be done, for example, on boot-up of the central controller 750. At step 704, the medical device controller 752 starts a user control device server and client for handling communications with other medical device controllers that are associated with controlling a medical device by a user control device. At step 706, the medical device controller 752 attempts to communicate with the central controller 750 via the communication port initialized by the central controller 750 at step 702 (such as using a predetermined port information saved in memory of the medical device controller 752). At step 708, the central controller 750 acknowledges the communication attempt from the medical device controller 752, dedicates another port for communication, and assigns an IP address to the medical device controller 752. The medical device controller 752 processes the IP address assignment at step 710.

At step 712, the central controller 750 updates its list of connected medical device controllers to include the medical device controller 752. At step 714, the central controller 750 transmits a request to the medical device controller 752 requesting its IP information. At step 716, the medical device controller 752 receives the request from the central controller 750 and replies with its IP information. At step 718, the central controller 750 transmits a request to all connected medical device controllers to update their list of available medical device controllers. The request may include the IP information of the medical device controller 752. The request may include the IP information for all connected medical device controllers with which it has established communication. At step 720, the medical device controller 752 updates its list of available medical device controller with the IP information transmitted by the central controller 750 at step 718. If the IP information includes an IP address for at least one medical device controller other than itself, then at step 722, the medical device controller 752 establishes a connection with the other medical device controller(s), enabling the medical device controller 752 and the other medical device controller(s) to communicate with one another.

At step 724, the central controller 750 subscribes to properties of the medical device controller 752. The properties can include available user control devices, which provides the central controller 750 information (e.g., an identifier) associated with any user control devices connected to the medical device controller 752. The properties can also include available medical devices, which provides the central controller 750 information (e.g., an identifier) associated with any medical devices connected to the medical device controller 752. At step 726, the medical device controller 752 processes the subscription request from the central controller 750 and provides the property information to the central controller 750 and provides a subscription token to the central controller 750 to confirm the subscription. Any changes to the properties over time may be notified to the central controller 750 via the subscription.

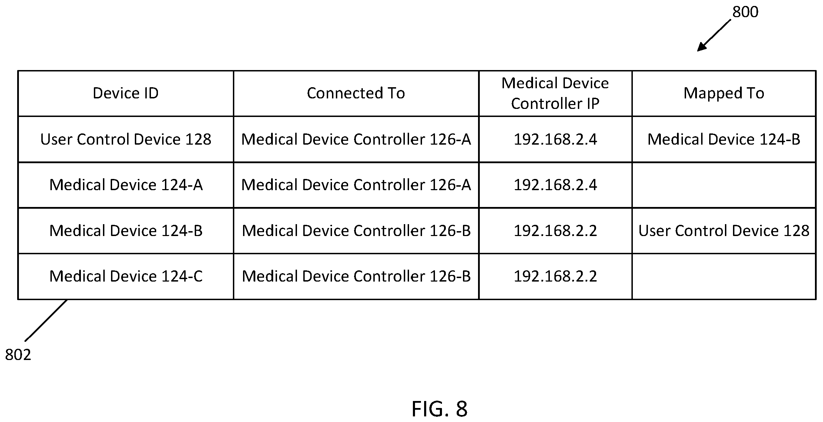

The central controller 750 may maintain a mapping table that includes information for the user control devices and medical devices connected to the medical device controllers, including medical device controller 752. FIG. 8 illustrates an exemplary mapping table 800 that may be maintained by the central controller 750. The table 800 may include a column 802 of device identifiers, which may include entries for each available user control device and medical device. For each device identifier, the table 800 may include an identifier for the medical device controller to which the corresponding device is connected and/or an IP address of the medical device controller to which the corresponding device is connected. The table 800 may include a mapping column that indicates which user control device is connected to which medical device. Table 800 is provided with exemplary entries associated with system 100 of FIG. 1. The column of device ID's includes user control device 128 and medical devices 124-A, 124-B, and 124-C. User control device 128 is connected to medical device controller 126-A, so medical device controller 126-A and its IP address (e.g., 192.168.2.4) is provided in the row for user control device 128. In the illustrated example, user control device 128 is mapped to medical device 124-B. Medical device 124-A is also connected to medical device controller 126-A. So, its row includes entries for medical device controller 126-A and the IP address for medical device controller 126-A. Medical devices 124-B and 124-C are connected to medical device controller 126-B and, thus, their respective rows include entries for medical device controller 126-B and its IP address (e.g., 192.168.2.2).

Method 700 may include updating mapping of one or more user control devices to one or more medical devices connected to one or more medical device controllers. Returning to FIG. 7A, at step 730, the central controller 750 may determine a mapping of a user control device connected to the medical device controller 752 or another medical device controller to a medical device connected to the medical device controller 752 or another medical device controller, such as using method 300 of FIG. 3. The central controller 750 may update a mapping table, such as mapping table 800 of FIG. 8, based on the determined mapping. At step 732, the central controller 750 may send an updated mapping to connected medical device controllers, including medical device controller 752. For example, the central controller 750 may send the mapping table 800 to the medical device controllers. At step 734, medical device controller 752 updates a mapping stored locally at the medical device controller 752. For example, medical device controller 752 may store mapping table 800 and may update it when updated mapping information is received from the central controller 750. Optionally, a user interface associated with the central controller 750 and/or a user interface associated with the medical device controller 752 may be updated to reflect the updated mapping at step 736.

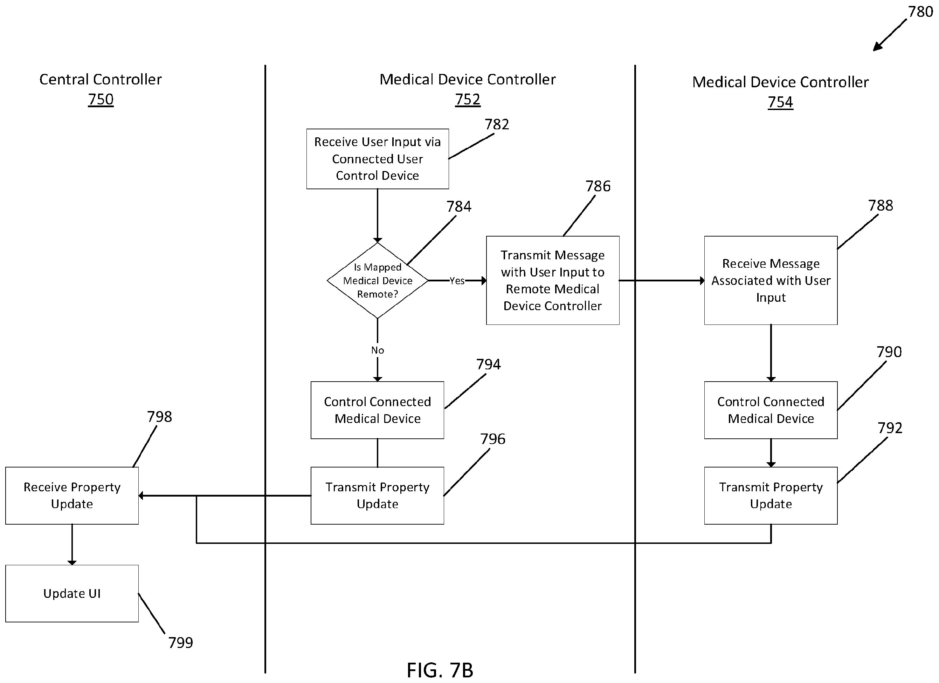

FIG. 7B illustrates an exemplary method 780 for using a user control device connected to medical device controller 752 to control a medical device connected to a remote medical device controller 754. Method 780 is an example of method 200 of FIG. 2. At step 782, medical device controller 752 receives a user input via a user control device connected to medical device controller 752. For example, the user control device may be a foot pedal and medical device controller 752 may receive a signal or data from the foot pedal associated with a percentage of press of the foot pedal. At step 784, medical device controller 752 determines whether the user control device is mapped to a medical device that is connected to medical device controller 752 or a medical device that is connected to a remote medical device controller. For example, medical device controller 752 may access a locally stored mapping table, such as mapping table 800 of FIG. 8, to determine the medical device and/or medical device controller to which the user control device is mapped. If the user control device is mapped to a medical device that is connected to remote medical device controller 754, medical device controller 752 may send a message, at step 786, to remote medical device controller 754 that includes information associated with the user input (e.g., a percentage press of the foot pedal). The message may be sent, for example, to the IP address of medical device controller 754.

At step 788, medical device controller 754 may receive the message from medical device controller 752, and at step 790, medical device controller 754 may control the medical device accordingly. For example, a speed of a cutting tool connected to medical device controller 754 may be increased to a level corresponding to the percentage press of the foot pedal. Optionally, at step 792, medical device controller 754 may transmit a property update associated with the control of the medical device to the central controller 750 (e.g., based on the property subscription established at step 726 of FIG. 7A). For example, medical device controller 754 may provide the speed of the cutting tool to the central controller 750.

Returning to step 784, if medical device controller 752 determines that the user control device is mapped to medical device controller 752, medical device controller 752 may control the medical device accordingly at step 794. Optionally, at step 796, medical device controller 752 may transmit a property update associated with the control of the medical device to the central controller 750. At step 798, the central controller 750 receive the property update from either medical device controller 752 or medical device controller 754 and may store the updated property information in a local memory. At step 799, central controller 750 may update a user interface to reflect the updated property information (for example, may update an indication of the speed of the cutting tool). Optionally, the user interface with the updated property information may include (or be displayed along with) surgical images or video such that a user is provided with the updated property information without the user having to turn away from observation of the surgical images or video.

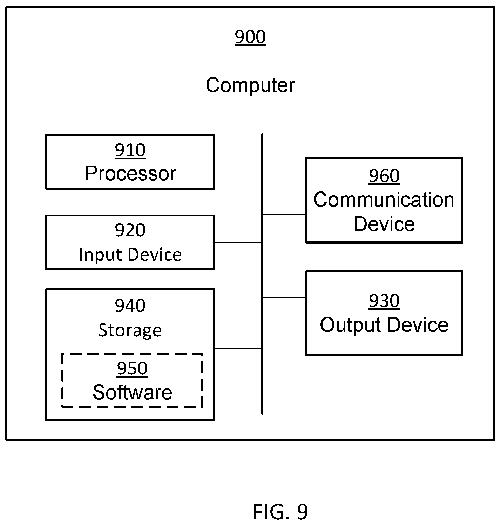

FIG. 9 illustrates an example of a computing system 900, in accordance with some examples of the disclosure. Computing system 900 can be used to implement one or more components of system 100 of FIG. 1, such as one or more of central controller 116, camera controller unit 112, remote computing system 150, medical device controller 126, etc. Computing system 900 can be a client or a server. As shown in FIG. 9, computing system 900 can be any suitable type of processor-based system, such as a personal computer, workstation, server, handheld computing device (portable electronic device) such as a phone or tablet, or dedicated device. The computing system 900 can include, for example, one or more of input device 920, output device 930, one or more processors 910, storage 940, and communication device 960. Input device 920 and output device 930 can either be connectable or integrated with computing system 900.

Input device 920 can be any suitable device that provides input, such as a touch screen, keyboard or keypad, mouse, gesture recognition component of a virtual/augmented reality system, or voice-recognition device. Output device 930 can be or include any suitable device that provides output, such as a display, touch screen, haptics device, virtual/augmented reality display, or speaker.

Storage 940 can be any suitable device that provides storage, such as an electrical, magnetic, or optical memory including a RAM, cache, hard drive, removable storage disk, or other non-transitory computer readable medium. Communication device 960 can include any suitable device capable of transmitting and receiving signals over a network, such as a network interface chip or device. The components of the computing system 900 can be connected in any suitable manner, such as via a physical bus or wirelessly.

Processor(s) 910 can be any suitable processor or combination of processors, including any of, or any combination of, a CPU, FPGA, and ASIC. Software 950, which can be stored in storage 940 and executed by one or more processors 910, can include, for example, the programming (e.g., one or more programs) that provides the functionality or portions of the functionality of the present disclosure (e.g., as described with respect to in the systems and methods as described above).

Software 950 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions to perform any of the methods, steps, and functions described herein. In the context of this disclosure, a computer-readable storage medium can be any medium, such as storage 940, that can contain or store programming for use by or in connection with an instruction execution system, apparatus, or device.

Software 950 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport computer readable medium can include, but is not limited to, an electronic, magnetic, optical, electromagnetic, or infrared wired or wireless propagation medium.

Computing system 900 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Computing system 900 can implement any operating system suitable for operating on the network. Software 950 can be written in any suitable programming language, such as C, C++, Java, or Python. In various aspects, application software providing the functionality of the present disclosure can be deployed in different configurations, such as in a client/server arrangement or through a Web browser as a Web-based application or Web service, for example.

The foregoing description, for the purpose of explanation, has been described with reference to specific example. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for controlling medical devices comprising:
receiving a user input to a user control device that is communicatively connected to a first medical device controller, the first medical device controller configured to control a first medical device;
transmitting a message associated with the user input from the first medical device controller to a second medical device controller; and
controlling, by the second medical device controller, based on the message, a second medical device communicatively connected to the second medical device controller.

2. The method of claim 1 comprising, prior to receiving the user input, associating the user control device with the second medical device, optionally wherein associating the user control device with the second medical device comprises receiving a user selection to associate the user control device with the second medical device.

3. The method of claim 2, wherein the user selection is received via the user control device.

4. The method of claim 2 or claim 3, wherein the user selection comprises a voice command, a gesture, an eye-tracking input, a virtual or augmented reality control, a button press, or an input to a touch input device.

5. The method of any of claims 2-4, wherein associating the user control device with the second medical device comprises determining use of the second medical device by a user.

6. The method of claim 5, wherein the use of the second medical device is determined based on video or image data.

7. The method of claim 6, wherein determining use of the second medical device by the user comprises identifying the second medical device in the video or image data.

8. The method of claim 7, wherein the video or image data comprises endoscopic video or image data capturing a surgical cavity within which the second medical device is positioned.

9. The method of claim 7, wherein the video or image data captures the user holding the second medical device.

10. The method of any of claims 5-9, wherein the use of the second medical device is determined based on sensor data generated by at least one sensor of the second medical device.

11. The method of any of claims 2-10, wherein associating the user control device with the second medical device comprises transmitting from a central controller to at least the first medical device controller at least one instruction associating the user control device with the second medical device.

12. The method of any of the preceding claims, comprising displaying on at least one display an indication that the user control device is set to control the second medical device.

13. The method of claim 12, comprising displaying the indication that the user control device is set to control the second medical device along with endoscopic video or images.

14. A system for controlling medical devices, the system configured for performing the method of any of claims 1-13.

15. A non-transitory computer readable storage medium storing instructions for execution by one or more processors of first and second medical device controllers to cause the first and second medical device controllers to :
perform the method of any of claims 1-13.
